# FASCICULE DE BREVET EUROPEEN

(11) **EP 2 991 569 B1**
(45) Date de publication et mention de la délivrance du brevet: **04.07.2018**
(21) Numéro de dépôt: 14727609.1
(22) Date de dépôt: 02.05.2014
(51) Int. Cl.: A61B 17/86, A61B 17/70

(54) **SYSTEME D'ANCRAGE OSSEUX POLYAXIAL A CHARGEMENT PAR LE DESSOUS**
VON UNTEN LADBARES POLYAXIALES KNOCHENVERANKERUNGSSYSTEM
BOTTOM-LOADING POLYAXIAL BONE ANCHORING SYSTEM

(30) Priorité: 02.05.2013 FR 1354053
(43) Date de publication de la demande: 09.03.2016
(73) Titulaire: Spinevision, 92160 Antony (FR)
(72) Inventeur: BAZILLE, Julien, F-91300 Massy (FR)
(74) Mandataire: IP Trust
(86) Numéro de dépôt international: PCT/FR2014/051051
(87) Numéro de publication internationale: WO 2014/177819

(56) Documents cités:
- WO-A1-2008/119006
- US-A1- 2007 135 817
- US-A1- 2012 179 212
- US-B2- 7 947 065

## Description

### DOMAINE TECHNIQUE DE L'INVENTION

L'invention concerne un système d'ancrage osseux polyaxial destiné à la correction et la stabilisation de vertèbres d'une colonne vertébrale.

Plus particulièrement, l'invention concerne un système d'ancrage osseux polyaxial à chargement par le dessous.

### ETAT DE LA TECHNIQUE

De manière classique, les systèmes d'ancrage vertébral comprennent des éléments d'ancrage osseux, tel que des vis, comprenant respectivement une tige filetée destinée à être fixé dans le pédicule d'une vertèbre et pourvue à l'une de ses extrémités d'un connecteur permettant de coupler l'élément d'ancrage osseux à un élément de stabilisation, tel qu'une tige de liaison.

Dans les montages complexes comprenant plusieurs segments rachidiens, il est répandu d'utiliser des systèmes d'ancrage osseux articulés à la jonction des éléments d'ancrage osseux et des connecteurs. On parle de système d'ancrage osseux polyaxial ou de vis pédiculaire polyaxiale. Cela permet ainsi d'ajuster la position des systèmes d'ancrage osseux et ainsi faciliter le montage des tiges de liaison dans ces derniers.

Afin d'offrir une plus grande modularité dans le type et le dimensionnement des éléments d'ancrage osseux et des connecteurs à assembler, il a été développé des systèmes d'ancrage osseux permettant le chargement par le dessous des éléments d'ancrage osseux avec les connecteurs.

Parmi ce type de systèmes d'ancrage osseux, il peut être cité celui décrit dans le brevet US7947065. Le système d'ancrage osseux décrit comprend un ensemble de connexion comprenant un connecteur formé d'un membre de connexion supérieur et d'un membre de connexion inférieur, d'une bague de retenue supérieur logée en partie inférieure du membre de connexion supérieure et d'une bague de retenue fendue logée dans le membre de connexion inférieur du connecteur. La surface inférieure du membre de connexion supérieure comporte une pluralité de rampes agencée pour coopérer avec la bague fendue inférieure durant l'assemblage de la vis avec le connecteur. Ces rampes permettent d'ouvrir la bague fendue de manière à laisser passer la tête sphérique de la vis au travers de la bague fendue. Ainsi, lorsque l'ensemble de connexion est monté sur la tête de vis, la bague fendue est poussée par la tête de la vis pour venir en contact avec la bague de retenue supérieur lequel comprime un ressort pour provoquer la mise en contact de la bague fendue inférieure avec les rampes, causant alors l'ouverture de la bague fendue pour permettre le passage de la tête de vis au travers de ladite bague. Une fois la tête de vis passée, le ressort entraine la bague fendue vers la partie basse du membre de connexion inférieur, empêchant la tête de vis de sortir de l'ensemble de connexion.

D'autres exemples de systèmes d'ancrage sont décrits également dans les demandes US2012/0179212 et WO2008/119006.

Le système de connexion du brevet US7947065, mais de manière générale les systèmes d'ancrage osseux à chargement par le dessous de l'état de la technique, présente l'inconvénient d'être relativement complexe. Il requiert en effet la mise en oeuvre de plusieurs éléments pour permettre l'insertion et la retenue de la tête de vis dans le connecteur, tel qu'un ressort, deux moyens de retenue et un système de rampes.

L'invention vise à remédier à ces problèmes en proposant un système d'ancrage osseux permettant le chargement par le dessous de l'élément d'ancrage osseux dans le connecteur de manière simple et rapide tout en assurant une tenue satisfaisante de l'élément d'ancrage osseux dans le connecteur.

### OBJET DE L'INVENTION

La présente invention décrivant un système d'ancrage osseux polyaxial est définie par la revendication 1 tandis que des modes de réalisation préféré sont exposés dans les revendications dépendantes.

A cet effet, l'invention propose un système d'ancrage osseux polyaxial comprenant un élément d'ancrage osseux pourvu d'une tête hémisphérique et un fut tubulaire pour le couplage d'un élément de stabilisation à l'élément d'ancrage osseux, ledit fut tubulaire comportant une embase traversée par un canal axial d'une section supérieure à la section de la tête hémisphérique, ledit canal axial présentant, en partie inférieure, un logement tronconique s'élargissant en direction opposée à la tête hémisphérique et, en amont du logement tronconique, un épaulement transversal d'une section intérieure sensiblement égale à la section de la tête hémisphérique, un moyen de blocage de l'élément d'ancrage osseux dans le fut tubulaire, ledit moyen de blocage étant placé dans le canal axial de l'embase, en amont du logement tronconique et une bague fendue conique de surface extérieure conjuguée à la surface intérieure du logement tronconique et de section minimale au repos inférieure à la section nominale de la tête hémisphérique, ladite bague étant réalisée en un matériau déformable entre une position expansée permettant le passage de la tête hémisphérique au travers de la bague, et une position rétrécie à une section inférieure à la section minimale au repos pour son insertion dans le logement tronconique, le logement tronconique présentant une hauteur supérieure à la hauteur de la bague fendue et, au voisinage de l'épaulement, une section supérieure à la section extérieure de la bague.

De part cette configuration et le dimensionnement de l'embase et de la bague, l'assemblage de l'élément d'ancrage osseux et du fut de couplage est réalisé uniquement par un déplacement conjoint de la bague fendue dans le logement tronconique et de la tête hémisphérique dans le canal axial.

Selon l'invention, le logement tronconique comporte, au voisinage de l'épaulement, une gorge radiale. Cela permet ainsi d'assurer un espace suffisant pour permettre l'expansion de la bague lors de sa traversée par la tête hémisphérique.

Avantageusement, le canal axial de l'embase comporte, au voisinage de l'épaulement une section élargie. Cela permet ainsi de faciliter l'insertion de la tête hémisphérique dans le canal axial.

Selon une configuration particulière, le moyen de blocage comprend un corps de berceau présentant une surface supérieure conformée pour recevoir la tige de liaison et une surface inférieure conformée pour recevoir la tête hémisphérique.

Avantageusement, le canal axial de l'embase est agencé avec le moyen de blocage pour permettre un déplacement axial du moyen de blocage dans le canal, entre une position dans laquelle le fut tubulaire est libre en rotation sur la tête hémisphérique de l'élément d'ancrage osseux et une position dans laquelle le fut tubulaire est bloqué sur la tête hémisphérique.

Avantageusement, le système d'ancrage comporte des moyens de guidage du moyen de blocage entre une position dans laquelle le fut tubulaire est libre en rotation sur la tête hémisphérique de l'élément d'ancrage osseux et une position dans laquelle le fut tubulaire est bloqué sur la tête hémisphérique. Selon un mode de réalisation particulier, le moyen de blocage comprend un corps de berceau prolongé par deux pattes s'étendant vers l'extérieur et destinées à être logées respectivement dans une gorge ménagée sur la paroi interne de l'embase, lesdites pattes et gorges formant les moyens de guidage du moyen de blocage.

Selon un mode de réalisation particulier, le fut de couplage présente deux évidements latéraux en forme de U pour recevoir une tige de liaison et un canal axial médian permettant l'engagement d'un bouchon de verrouillage du système d'ancrage osseux, le canal axial médian débouchant dans le canal axial de l'embase.

### BREVE DESCRIPTION DES FIGURES

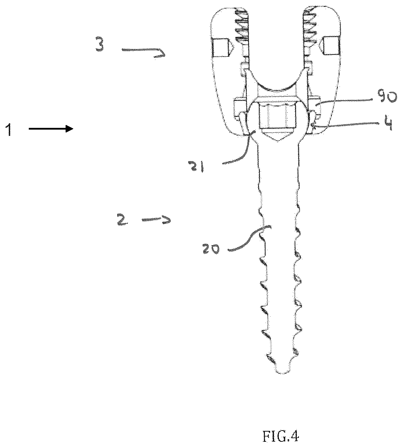

D'autres objets et avantages de l'invention apparaîtront au cours de la description qui suit, faite en référence aux dessins annexés, l'invention étant représentée par la figure 4, les autres figures illustrant des exemples de réalisation utiles à la compréhension de l'invention.

Plus particulièrement :
- la figure 1 représente une vue partielle en coupe longitudinale d'un système d'ancrage osseux;
- la figure 2 représente une vue éclatée du système d'ancrage osseux de la figure 1 ;
- les figures 3a à 3e représentent les étapes d'assemblage des éléments constitutifs du système d'ancrage osseux de la figure 1 ;
- la figure 4 représente une vue en coupe longitudinale d'un système d'ancrage osseux selon un mode de réalisation de l'invention.

### DESCRIPTION DETAILLEE DES FIGURES

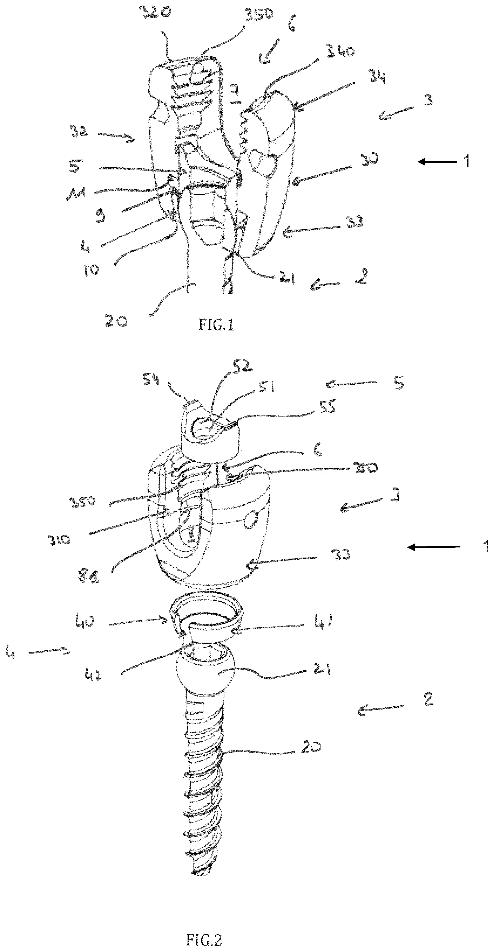

En relation avec les figures 1 et 2, il est décrit un système d'ancrage osseux 1 selon un premier mode de réalisation comprenant un élément d'ancrage osseux 2 destiné à venir s'implanter dans une vertèbre, un connecteur 3 se présentant sous la forme d'un fut tubulaire (on parlera alors de fut de couplage) ainsi qu'un moyen de retenue 4 de l'élément d'ancrage osseux 2 dans le connecteur 3 et un moyen de blocage 5 de l'élément d'ancrage osseux dans le connecteur 3 dans une position donnée.

L'élément d'ancrage osseux 2 présente un axe fileté 20 prolongé au niveau de l'une de ses extrémités par une tête hémisphérique 21. Dans ce qui suit, on désignera la tête hémisphérique par les termes « tête de vis 21 ».

Le fut de couplage 3 comporte un corps de tulipe 30 pourvu de deux bras 32, 34 et d'une embase 33.

Les deux bras sont agencés pour délimiter deux évidements latéraux 310, 330 en forme de U pour recevoir une tige de liaison (non représentée) ainsi qu'un premier canal axial médian 7 destiné à recevoir un élément de verrouillage (non représenté) du système d'ancrage osseux 1, du type écrou de compression. A cet effet, la face interne de chacun des bras 32, 34 présentent un filetage 350 apte à coopérer avec le filetage extérieur de l'écrou de compression. Les extrémités supérieures 320, 340 des bras 30, 32 délimitent une ouverture d'entrée 6 de l'écrou de compression.

L'embase 33 est traversée par un canal axial médian 8 désigné par la suite de deuxième canal axial par opposition au canal axial médian de la partie supérieure désigné quant à lui de premier canal axial. Le deuxième canal 8, lequel débouche dans le premier canal 7, présente, en extrémité inférieure de l'embase 33, une ouverture d'entrée 10 pour permettre le passage de la tête de vis 21 dans le corps de tulipe 30. Pour des raisons qui seront explicitées plus loin, le deuxième canal 8 présente une section supérieure à la section de la tête de vis 21.

Avantageusement, le deuxième canal 8 comporte, en partie inférieure, un logement 9 de forme tronconique s'élargissant dans une direction opposée à la tête de vis 21. Comme on le verra plus loin, le logement tronconique 9 est destiné à recevoir le moyen de retenue (bague fendue) et la tête de vis 21.

En amont du logement tronconique 9, c'est-à-dire en s'éloignant de la tête de vis 21 lorsque celle-ci est montée dans le fut de couplage 3, le deuxième canal 8 comporte un épaulement transversal 11 délimitant la partie haute du logement tronconique 9. L'épaulement 11 présente avantageusement une section déterminée pour laisser passer la tête de vis 21 dans la partie haute du deuxième canal 8 tout en empêchant le passage du moyen de retenue. Dans le mode de réalisation décrit, l'épaulement 11 est sensiblement égal à la section de la tête de la vis 21.

Dans ce qui suit, la partie du corps tubulaire 30 délimitée par le filetage interne 350 des bras 32, 34 caractérise la partie supérieure du fut de couplage 3 (ou du corps tubulaire 30). De même, la partie de l'embase 33 délimitée par le logement tronconique 9 caractérise la partie inférieure du fut de couplage 3 (ou du corps tubulaire 30) tandis que la partie de l'embase 33 s'étendant entre la partie supérieure et la partie inférieure du fut de couplage 3 caractérise la partie intermédiaire du fut de couplage 3.

Comme on le verra plus loin, la partie intermédiaire est destinée à recevoir le moyen de blocage 5 de la tête de vis 21 en position par rapport au fut de couplage 3 ainsi que, durant l'assemblage de l'élément d'ancrage osseux avec le fut de couplage 3, la tête de vis 21, tandis que la partie inférieure est destinée à recevoir le moyen de retenue de la tête vis 21 dans le fut de couplage 3.

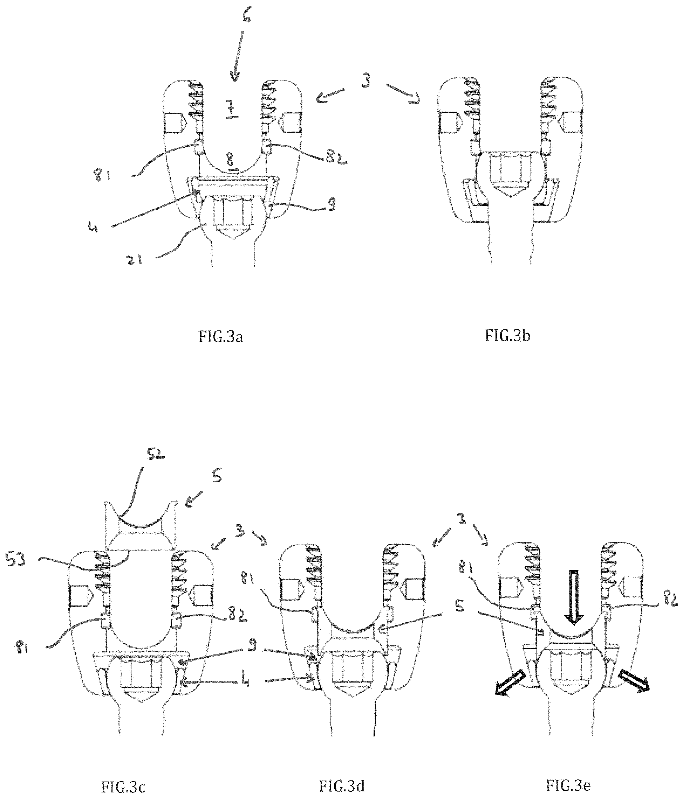

Dans l'exemple illustré, la partie intermédiaire comprend en partie haute, dans le prolongement des bras 32, 34, deux gorges 81, 82 ménagées dans la paroi interne de l'embase 33. Les gorges 81, 82 permettent le guidage du moyen de blocage entre une position dans laquelle le mouvement du fut de couplage 3 sur la tête de vis 21 est autorisé et une position dans laquelle le mouvement du fut de couplage 3 sur la tête de vis 21 est empêché (position de blocage) comme illustré sur les figure 3d et 3e et décrit plus loin. Avantageusement, la partie intermédiaire présente en entrée une section élargie (non représentée) afin de faciliter l'insertion de la tête de vis 21 dans cette dernière.

Selon l'invention, le moyen de retenue se présente sous la forme d'une bague 4 conique fendue transversalement, conformée pour présenter une surface extérieure conjuguée à la surface intérieure du logement tronconique 9 de la partie inférieure du fut de couplage 3.

Avantageusement, la bague fendue 4 est réalisée dans un matériau déformable. Le matériau est choisi de telle manière que la bague fendue 4 est déformable entre une position expansée permettant le passage de la tête de vis 21 au travers de ladite bague et une position rétrécie dans laquelle la bague fendue 4 présente une section permettant son insertion dans le logement tronconique 9. Plus particulièrement, la bague fendue 4 devra présenter, en position rétrécie, une section permettant son passage au travers de l'ouverture d'entrée 10 inférieure du fut de couplage 3. Par ailleurs, la bague fendue 4 est conformée pour présenter au repos une section minimale inférieure à la section nominale de la tête de vis 21.

Avantageusement, la bague fendue 4 présente une hauteur inférieure à la hauteur du logement tronconique 9. Cet agencement a pour avantage de permettre, comme on le verra plus loin, le déplacement de la bague fendue 4 à l'intérieur du logement tronconique 9, la bague fendue 4 étant mobile entre une position haute permettant le passage de la tête de la vis à travers la bague fendue 4 et une position basse dans laquelle la tête de la vis est retenue dans le logement tronconique 9, en partie basse du fut de couplage.

Le moyen de blocage 5 de la tête de vis 21 dans le fut de couplage 3 consiste en une pièce formant berceau, appelée par la suite berceau de blocage 5, destinée à être placée entre la tête de vis 21 et la tige de liaison lorsque celle-ci est placée dans le système d'ancrage osseux 1. Dans le mode de réalisation décrit, le berceau de blocage 5 comporte un corps de berceau 50 pourvu d'un alésage axial 51 traversant et présentent une surface de contact supérieure 52 de forme complémentaire à celle de la surface extérieure de la tige de liaison et une surface de contact inférieure 53 de forme complémentaire à celle de la tête de vis 21.

Le corps de berceau 50 présente un diamètre extérieur légèrement inférieur à celui du deuxième canal axial 8.

Avantageusement, le corps de berceau 50 est prolongé par deux pattes 54, 55 s'étendant vers l'extérieur, ie. dans une direction opposée à l'alésage 51 du corps de berceau. Les pattes 54, 55 sont destinées à venir se loger respectivement dans une des gorges 81, 82 ménagées dans la paroi interne de l'embase 33. Les pattes 54, 55 constituent avec les gorges 81, 82 des moyens de guidage du berceau de blocage 5 entre la position dans laquelle le fut de couplage 3 est libre en rotation sur la tête de vis 21 et la position de blocage dans laquelle le fut de couplage 3 est bloqué sur la tête de vis 21.

Avantageusement, les gorges 81, 82 sont dimensionnées de manière à laisser un jeu axial au berceau de blocage 5 lorsque celui-ci est monté dans la partie intermédiaire. Le corps de berceau 50 peut ainsi être déplacé légèrement vers le haut ou vers le bas lorsqu'il est monté dans le fut de couplage 3. Comme on le verra plus loin, ce jeu permet au berceau de couplage 5 de passer d'une position dans laquelle le fut de couplage 3 est libre en rotation sur la tête de vis 21 à une position bloquée dans laquelle le fut de couplage 3 est bloqué en position sur la tête de vis 21, tout mouvement de rotation étant empêché.

Avantageusement, la surface extérieure du corps de berceau 50 destinée à recevoir la tige de liaison peut présenter des stries, des rugosités, des cannelures ou similaires afin d'augmenter la prise par frottement entre le corps de berceau 50, la tige de liaison et le moyen de verrouillage lorsque celui-ci est placé dans le premier canal axial du fut de couplage 3. De la même manière, il peut être prévu une tige présentant des stries, des rugosités, des cannelures ou similaires afin d'améliorer la prise par frottement entre lesdits éléments.

Les gorges 81, 82 et le corps de berceau 50 sont dimensionnés pour que lorsque le corps de berceau 50 est en position de blocage, le corps du berceau 50 présente une partie s'étendant dans le logement tronconique 9 de manière à venir en contact avec la tête de vis 21.

L'élément d'ancrage osseux 2 est assemblé avec le fut de couplage 3 de la manière suivante.

La première étape consiste à clipser la bague fendue 4 dans le logement tronconique 9. Pour ce faire, et selon une technique de clipsage choisie parmi d'autres, la bague 4 est disposée par rapport au fut de couplage 3 de manière à présenter sa fente 42 en vis-à-vis de l'ouverture d'entrée inférieure 10. Un effort de compression est ensuite exercé sur la bague fendue 4 afin de rapprocher les deux bras 40, 41 de la bague fendue 4 l'un vers l'autre et placer la bague fendue 4 dans la position rétrécie permettant son passage au travers de l'ouverture 10. La bague 4 est alors poussée dans le logement tronconique 9. Une fois partiellement insérée dans celui-ci, la bague fendue 4 est soumise à un mouvement de basculement pour être amenée dans le bon sens, ie. placée de manière à présenter la partie la plus large à l'opposé de l'ouverture d'entrée inférieure 10. Une fois placée dans le logement tronconique 9, la bague fendue 4 reprend sa forme au repos.

Une fois la bague fendue 4 en place dans le logement tronconique 9, l'élément d'ancrage est clipsé dans le fut de couplage 3. Pour ce faire, la tête de vis 21 est présentée devant l'ouverture d'entrée inférieure 10 et insérée dans le logement tronconique 9. La section nominale de la tête de vis 21 étant supérieure à la section minimale interne de la bague au repos, la tête de vis 21 entraîne la bague fendue 4 vers le haut du logement tronconique 9 jusqu'à ce que cette dernière vienne en contact avec l'épaulement 11 (figure 3a). Le mouvement de translation de la tête de vis 21 en direction de l'ouverture d'entrée 6 de l'écrou de compression étant poursuivi, la tête de vis 21 pénètre dans le deuxième canal 8 destiné à recevoir le berceau de couplage 5. Les bras 40, 41 de la bague fendue 4, du fait de son blocage par l'épaulement 11, s'écartent l'un de l'autre sous l'effort de poussée de la tête de vis 21, jusqu'à prendre une position expansée permettant le passage de la tête de vis 21 au travers de ladite bague fendue 4. Une fois la tête de vis 21 passée dans le deuxième canal, la bague fendue 4 reprend sa position au repos dans le logement tronconique 9 (figure 3b).

L'élément d'ancrage osseux 2 est alors tiré en sens inverse, en direction de l'ouverture d'entrée 10 de la tête de vis 21 de manière à ramener la tête de vis 21 dans le logement tronconique 9. Lors de son mouvement, la tête de vis 21 entraine avec elle la bague fendue 4, alors en position de repos, en direction de l'ouverture d'entrée inférieure pour parvenir à la position de maintien de la tête de vis 21 dans le fut de couplage 3 comme illustré sur la figure 3c. Il est à noter qu'une fois assemblé avec le fut de couplage 3, l'élément d'ancrage osseux 2 ne peut plus être démonté sans casser l'un des éléments.

L'étape qui suit est le clipsage du berceau de couplage 5 dans le fut de couplage 3. Pour ce faire, le berceau de couplage 5 est présenté devant l'ouverture d'entrée supérieure 6 puis est glissé à l'intérieur du fut de couplage 3 pour être placé dans le deuxième canal, en partie intermédiaire du fut de couplage 3 (figure 3c et 3d).

Comme décrit plus haut, le berceau de blocage peut se déplacer axialement dans le deuxième canal axial. Lorsque le berceau de couplage 5 est placé en position haute dans le deuxième canal médian, comme illustré sur la figure 3d, un jeu est laissé entre la surface inférieure du berceau et la tête de vis 21 de sorte que la tête de vis 21 peut pivoter dans le fut de couplage 3. Lorsque le berceau est placé en position basse, le berceau est plaqué contre la tête de la vis, empêchant ainsi tout mouvement de la tête de vis 21 dans le fut de couplage 3, la bague fendue 4 exerçant de son côté des efforts latéraux empêchant toute expansion de cette dernière. En d'autres termes, tout mouvement de la tête de vis 21 est empêché par la bague et le berceau. La figure 3e illustre les efforts s'exerçant entre les éléments pour bloquer la multi-axialité de la tête de vis 21.

Ainsi, une fois l'élément d'ancrage osseux 2 assemblé avec le fut de couplage 3 et le berceau en place,

Selon une configuration particulière illustrée sur la figure 4, la partie inférieure comporte avantageusement une gorge interne 90 s'étendant radialement. La présence d'une telle gorge autorise une expansion plus importante du moyen de retenue lors du passage de la tête de vis 21. La mise en en place de la tête de vis 21 dans le fut de couplage 3 s'en trouve ainsi facilitée, y compris pour des têtes de vis de dimension relativement large.

L'invention est décrite dans ce qui précède à titre d'exemple. Il est entendu que l'homme du métier est à même de réaliser différentes variantes de réalisation de l'invention sans pour autant sortir du cadre de l'invention.

## Revendications

1. Système d'ancrage osseux polyaxial (1) comprenant :
- un élément d'ancrage osseux pourvu d'une tête hémisphérique (21),
- un fut tubulaire (3) pour le couplage d'un élément de stabilisation à l'élément d'ancrage osseux, ledit fut tubulaire (3) comportant une embase (33) traversée par un canal axial (8) d'une section supérieure à la section de la tête hémisphérique (21), ledit canal axial (8) présentant, en partie inférieure, un logement tronconique (9) s'élargissant en direction opposée à la tête hémisphérique (21) et, en amont du logement tronconique (9), un épaulement (11) transversal d'une section intérieure sensiblement égale à la section de la tête hémisphérique (21),
- un moyen de blocage (5) de l'élément d'ancrage osseux dans le fut tubulaire (3), ledit moyen de blocage étant placé dans le canal axial de l'embase (33), en amont du logement (9), et
- une bague fendue (4) conique de surface extérieure conjuguée à la surface intérieure du logement tronconique (9) et de section minimale au repos inférieure à la section nominale de la tête hémisphérique (21), ladite bague étant réalisée en un matériau déformable entre une position expansée permettant le passage de la tête hémisphérique (21) au travers de la bague fendue (4) et une position rétrécie à une section inférieure à la section minimale au repos pour son insertion dans le logement tronconique (9), le logement tronconique (9) présentant une hauteur supérieure à la hauteur de la bague fendue (4) et, au voisinage de l'épaulement (11), une section supérieure à la section extérieure de la bague,
**caractérisé en ce que** le logement tronconique (9) comporte, au voisinage de l'épaulement (11) lequel délimite la partie haute du logement tronconique (9), une gorge radiale (90).

2. Système d'ancrage osseux polyaxial (1) selon la revendication 1, **caractérisé en ce que** le canal axial de l'embase (33) comporte, au voisinage de l'épaulement (11) une section élargie (80).

3. Système d'ancrage osseux polyaxial (1) selon la revendication 1 ou la revendication 2, **caractérisé en ce que** le canal axial (8) de l'embase (33) est agencé pour permettre un déplacement axial du moyen de blocage (5) dans le canal (8), entre une position dans laquelle le fut tubulaire (3) est libre en rotation sur la tête hémisphérique (21) de l'élément d'ancrage osseux et une position dans laquelle le fut tubulaire (3) est bloqué sur la tête hémisphérique (21).

4. Système d'ancrage osseux polyaxial (1) selon l'une quelconque des revendications 1 à 3, **caractérisé en ce qu'**il comporte des moyens de guidage du moyen de blocage (5) entre une position dans laquelle le fut tubulaire (3) est libre en rotation sur la tête hémisphérique (21) de l'élément d'ancrage osseux et une position dans laquelle le fut tubulaire (3) est bloqué sur la tête hémisphérique (21).

5. Système d'ancrage osseux polyaxial (1) selon la revendication précédente, **caractérisé en ce que** le moyen de blocage comprend un corps de berceau (50) prolongé par deux pattes (54, 55) s'étendant vers l'extérieur et destinées à être logées respectivement dans une gorge ménagée sur la paroi interne de l'embase (33), lesdites pattes et gorges formant les moyens de guidage du moyen de blocage (5).

6. Système d'ancrage osseux polyaxial (1) selon l'une quelconque des revendications 1 à 5, **caractérisé en ce que** le moyen de blocage comprend un corps de berceau (50) présentant une surface supérieure conformée pour recevoir la tige de liaison et une surface inférieure conformée pour recevoir la tête hémisphérique (21).

7. Système d'ancrage osseux polyaxial (1) selon l'une quelconque des revendications 1 à 6, **caractérisé en ce que** le fut de couplage (3) présente deux évidements latéraux en forme de U pour recevoir un tige de liaison et un canal axial médian (7) permettant l'engagement d'un bouchon de verrouillage du système d'ancrage osseux, le canal axial médian (7) débouchant dans le canal axial (8) de l'embase (33).

## Patentansprüche

1. Polyaxiales Verankerungssystem (1) bestehend aus:
- einem Knochenverankerungselement, das mit einem halbkugelförmigen Kopf (21) versehen ist,
- einem rohrförmigen Schaft (3) zum Verbinden eines Stabilisierungselements mit dem Knochenverankerungselement, wobei besagter rohrförmiger Schaft (3) einen Sockel (33) enthält, durch den ein axialer Kanal (8) verläuft, dessen Querschnitt größer ist, als der Querschnitt des halbkugelförmigen Kopfes (21), wobei besagter axialer Kanal (8) im unteren Teil eine kegelstumpfförmige Aufnahme (9) aufweist, die sich in die dem halbkugelförmigen Kopf (21) entgegengesetzte Richtung verbreitert, und vor der kegelstumpfförmigen Aufnahme (9) eine quer verlaufende Schulter (11), deren Innenquerschnitt im Wesentlichen gleich dem Querschnitt des halbkugelförmigen Kopfes (21) ist,
- einem Mittel zum Blockieren (5) des Knochenverankerungselements im rohrförmigen Schaft (3), wobei besagtes Blockierungsmittel vor der Aufnahme (9) in den axialen Kanal des Sockels (33) eingesetzt wird, und
- einer konischen Spannbuchse (4), deren Außenfläche die Gegenfläche zur Innenfläche der kegelstumpfförmigen Aufnahme (9) bildet, und deren Mindestquerschnitt in Ruhestellung kleiner ist, als der Nennquerschnitt des halbkugelförmigen Kopfes (21), wobei besagte Spannbuchse aus einem Material hergestellt ist, das sich von einer ausgedehnten Position, die das Durchführen des halbkugelförmigen Kopfes (21) durch die Spannbuchse (4) ermöglicht, in Ruhestellung zu einer eingezogenen Position verformen kann, die einen kleineren Querschnitt als den Mindestquerschnitt hat, um in die kegelstumpfförmige Aufnahme (9) geschoben werden zu können, wobei die kegelstumpfförmige Aufnahme (9) höher ist, als die Spannbuchse (4) und neben der Schulter (11) einen Querschnitt aufweist, der größer ist, als der Außenquerschnitt des Rings, **dadurch gekennzeichnet, dass** die kegelstumpfförmige Aufnahme (9) neben der Schulter (11), welche den oberen Teil der kegelstumpfförmigen Aufnahme (9) begrenzt, eine radiale Nut (90) aufweist.

2. Polyaxiales Verankerungssystem (1) nach Anspruch 1, **dadurch gekennzeichnet, dass** der axiale Kanal des Sockels (33) neben der Schulter (11) einen erweiterten Querschnitt (80) umfasst.

3. Polyaxiales Knochenverankerungssystem (1) nach Anspruch 1 oder 2, **dadurch gekennzeichnet, dass** der axiale Kanal (8) des Sockels (33) so angeordnet ist, dass die axiale Verschiebung des Sperrmittels (5) im Kanal (8) zwischen einer Position, in welcher der röhrenförmige Schaft (3) auf dem halbkugelförmigen Kopf (21) des Knochenverankerungselements frei drehbar ist, und einer Position, in welcher der röhrenförmige Schaft (3) auf dem halbkugelförmigen Kopf (21) blockiert ist, wechseln kann.

4. Polyaxiales Knochenverankerungssystem (1) nach einem der Ansprüche 1 bis 3, **dadurch gekennzeichnet, dass** es Mittel zur Führung des Blockierungsmittels (5) von einer Position, in welcher der röhrenförmige Schaft (3) auf dem halbkugelförmigen Kopf (21) des Knochenverankerungselements frei drehbar ist, in eine Position, in welcher der röhrenförmige Schaft (3) auf dem halbkugelförmigen Kopf (21) blockiert wird, aufweist.

5. Polyaxiales Knochenverankerungssystem (1) nach dem vorhergehenden Anspruch, **dadurch gekennzeichnet, dass** das Blockierungsmittel einen Wiegekörper (50) umfasst, der durch zwei sich nach außen erstreckende Ansätze (54, 55) verlängert wird, die dazu bestimmt sind, jeweils in einer an der Innenwand des Sockels (33) ausgebildeten Nut aufgenommen zu werden, wobei besagte Ansätze und Nuten die Führungsmittel des Blockierungsmittels (5) bilden.

6. Polyaxiales Knochenverankerungssystem (1) nach einem der Ansprüche 1 bis 5, **dadurch gekennzeichnet, dass** das Blockierungsmittel einen Wiegekörper (50) mit einer oberen Fläche aufweist, die so angeglichen ist, dass sie den Verbindungsstab aufnehmen kann, und eine untere Fläche, die so angeglichen ist, dass sie den halbkugelförmigen Kopf (21) aufnehmen kann.

7. Polyaxiales Knochenverankerungssystem (1) nach einem der Ansprüche 1 bis 6, **dadurch gekennzeichnet, dass** der Verbindungsschaft (3) zwei seitliche, u-förmige Aussparungen zur Aufnahme eines Verbindungsstabs aufweist, und einen mittleren axialen Kanal (7), der das Eingreifen einer Verschlusskappe des Knochenverankerungssystems ermöglicht, wobei der mittlere axiale Kanal (7) bis zum axialen Kanal (8) des Sockels (33) führt.

## Claims

1. A polyaxial bone anchoring system (1) comprising:
- a bone anchoring member provided with a hemispherical head (21),
- a tubular barrel (3) for coupling a stabilizing element to the bone anchoring member, with said tubular barrel (3) comprising a base (33) traversed by an axial channel (8), the cross section of which is greater than the cross section of the hemispherical head (21), with said axial channel (8) having, in the lower part, a frustoconical housing (9) which widens in the direction opposite the hemispherical head (21) and, upstream from the frustoconical housing (9), a transverse shoulder (11), the internal cross-section of which is substantially equal to the cross-section of the hemispherical head (21),
- means for locking (5) the bone anchoring member in the tubular barrel (3), with said locking means being positioned in the axial channel of the base (33), upstream from the housing (9), and
- a conical split ring (4) the outer surface of which matches the inner surface of the frustoconical housing (9) and the minimum cross-section of which, when idle, is smaller than the nominal cross-section of the hemispherical head (21), with said ring being made of a deformable material between an expanded position allowing the passage of the hemispherical head (21) through the split ring (4) and a retracted position, having a cross section smaller than the minimum cross-section, when idle, to be inserted into the frustoconical housing (9), with the frustoconical housing (9) having a height greater than the height of the split ring (4) and, close to the shoulder (11), a cross section greater than the outer cross section of the ring, **characterized in that** the frustoconical housing (9) has a radial groove (90), close to the shoulder (11), which delimits the upper part of the frustoconical housing (9).

2. A polyaxial bone anchoring system (1) according to claim 1, **characterized in that** the axial channel of the base (33) has a widened section (80), close to the shoulder (11) .

3. A polyaxial bone anchoring system (1) according to claim 1 or claim 2, **characterized in that** the axial channel (8) of the base (33) is so arranged as to allow an axial displacement of the locking means (5) in the channel (8), between a position in which the tubular barrel (3) is free to rotate on the hemispherical head (21) of the bone anchoring member and a position in which the tubular barrel (3) is locked on the hemispherical head (21).

4. A polyaxial bone anchoring system (1) according to any one of claims 1 to 3, **characterized in that** it comprises means for guiding the locking means (5) between a position in which the tubular barrel (3) is free to rotate on the hemispherical head (21) of the bone anchoring member and a position in which the tubular barrel (3) is locked on the hemispherical head (21).

5. A polyaxial bone anchoring system (1) according to the preceding claim, **characterized in that** the locking means comprises a cradle body (50) extended by two lugs (54, 55) extending outwardly and intended to be respectively housed in a groove provided on the inner wall of the base (33), with said lugs and grooves forming the means for guiding the locking means (5).

6. A polyaxial bone anchoring system (1) according to any one of claims 1 to 5, **characterized in that** the locking means comprises a cradle body (50) having an upper surface shaped for receiving the connecting rod and a lower surface shaped for receiving the hemispherical head (21).

7. A polyaxial bone anchoring system (1) according to any one of claims 1 to 6, **characterized in that** the coupling barrel (3) comprises two U-shaped lateral recesses to receive a connecting rod and a median axial channel (7) allowing the engagement of a locking plug of the bone anchoring system, with the median axial channel (7) opening into the axial channel (8) of the base (33).
